# EUROPÄISCHE PATENTSCHRIFT

(11) **EP 0 379 933 B1**
(45) Veröffentlichungstag und Bekanntmachung des Hinweises auf die Patenterteilung: **28.09.1994**
(21) Anmeldenummer: 90100806.0
(22) Anmeldetag: 16.01.1990
(51) Int. Cl.: A61L 15/16, A61K 9/70

(54) **Superfizielles therapeutisches System mit einem Gehalt an einem antineoplastischen Wirkstoff, insbesondere 5-Fluoruracil**
Surface treatment system containing an antineoplastic agent, particularly 5-fluor uracil
Système de thérapie en surface contenant un agent antinéoplaste, notamment le 5-fluoruracil

(30) Priorität: 20.01.1989 DE 3901551
(43) Veröffentlichungstag der Anmeldung: 01.08.1990
(73) Patentinhaber: LTS Lohmann Therapie-Systeme GmbH & Co. KG, 56513 Neuwied (DE)
(72) Erfinder: Müller, Walter, Dr., Dipl.-Chem., D-5450 Neuwied 1 (DE); Kindel, Heinrich, D-5455 Rengsdorf (DE)
(74) Vertreter: Flaccus, Rolf-Dieter, Dr.

(56) Entgegenhaltungen:
- EP-A- 0 155 229
- EP-A- 0 224 981
- EP-A- 0 261 402
- EP-A- 0 283 434
- EP-A- 0 297 769
- GB-A- 2 094 809
- US-A- 3 734 097

## Beschreibung

Die Erfindung betrifft ein superfizielles therapeutisches System mit einem antineoplastischen Wirkstoff, insbesondere 5-Fluoruracil, bestehend aus einer undurchlässigen Rückschicht, einer wirkstoffhaltigen Matrix und einer wiederablösbaren Schutzschicht.

Die Erfindung betrifft ein superfizielles therapeutisches System mit einem antineoplastischen Wirkstoff, insbesondere 5-Fluoruracil.

Cytostatisch und/oder cytotoxisch wirksame Substanzen spielen in der medizinischen Therapie eine Rolle, wo es darum geht, in überschießendes Zellwachstum regulierend einzugreifen. Die größte Anwendung finden sie deshalb in der Therapie von malignen Tumoren.

Lokal appliziert werden sie aber auch eingesetzt zur Therapie von weniger bedrohlichen Krankheiten wie Psoriasis, von Viren herrührenden Warzen, Keratosen, Morbus Bowen und oberflächennahen Basaliomen. Man erzielt dabei die für eine erfolgreiche Therapie notwendigen hohen lokalen Wirkstoffkonzentrationen, ohne die bei der systemischen Chemotherapie von malignen Tumoren auftretenden Nebenwirkungen, die den Therapieabbruch erzwingen können und vereinzelt auch zu Todesfällen führten, in Kauf nehmen zu müssen. Im Handel erhältlich sind zur lokalen Anwendung zwei Salben, die 5-Fluoruracil als Wirkstoff enthalten (Effudix und Effluderm, beide von Hoffmann La Roche AG).

5-Fluoruracil wird zu den sogenannten Antimetaboliten gezählt und ist speziell ein Pyrimidinantimetabolit,

Klinische Erfahrungen mit diesem Wirkstoff als topisches Cytostatikum liegen seit ca. 25 Jahren vor.

Er wird besonders geschätzt wegen seiner guten medizinischen und kosmetischen Resultate (Goette, D.K.; J. AM. ACAD. DERMATOL: 4: 633-649 1981)

Die Anwendung in Form von Salben hat aber den Nachteil, daß es schwierig, wenn nicht unmöglich ist, ein bestimmtes Hautareal über die gesamte Behandlungsdauer, die sich über mehrere Wochen erstrecken kann, mit einer einerseits ausreichenden, andererseits aber nicht überdosierten Menge Wirkstoff zu versorgen.

Dieser Nachteil wurde auch bereits erkannt und führte zu dem in der US-PS 3,734,097 beschriebenen Applikationssystem, bestehend aus einer selbstklebenden, flächenförmigen und wirkstoffhaltigen Formulierung, die auf der einen Seite mit einer für den Wirk- und die Hilfsstoffe undurchlässigen Trägerfolie und auf der anderen Seite mit einer über gleiche Eigenschaften verfügenden, zusätzlich aber vor Gebrauch entfernbaren, Abziehfolie versehen ist.

Vor dem gleichen Hintergrund ist die US-PS 3,769,071 zu sehen, bei der Polyurethane als inertes Trägermaterial für den Wirkstoff 5-Fluoruracil genommen wurden.

Aufgabe dieser Erfindung war es nun, auf dieser Basis ein System zu entwickeln, das alle Vorteile bisheriger Formulierungen besitzt, zusätzliche Verbesserungen aufweist und sich in praktischen Versuchen bewährt.

Während der Behandlung mit cytostatisch und cytotoxisch wirksamen Substanzen werden die Zellen mit einer erhöhten Teilungsaktivität graduell stärker geschädigt als sich normal teilende Zellen. Es ist eine erwünschte, und für die erfolgreiche Therapie notwendige Folge, daß es im Anwendungsbereich zu einem vermehrten Absterben von teilungsaktiven Zellen kommt. Begleitet ist dieses vermehrte Zellsterben von inflammatorischen Prozessen, die wiederum verbunden sind mit der Absonderung von Wundsekrekten. Diese erhöhte Wundsekretabsonderung macht es nun schwierig, ein bestimmtes Hautareal über längere Zeit unter Okklusionsbedingungen zu halten, ohne daß das System seine Haftung zur Haut verliert. Lösungen dieses Problems, wie das Vorsehen eines den wirkstoffhaltigen Teil des Systems überragenden selbstklebenden Randes sind dabei nicht als optimal anzusehen, da sie die Gesamtfläche des Systems vergrößern und deshalb die Applikation besonders im Gesichtsbereich erschweren.

Der Zusatz eines Wasserabsorbers in ein selbstklebendes Polyacrylat mit der Absicht, die Hauthaftung des letzteren zu verbessern, ist in EP-A-0 297 769 und GB-A-2 094 809 offenbart. Dabei beschreibt das erstere Dokument eine hydrophile adhäsive Komposition zur Verwendung auf der menschlichen Haut mit einer drucksensitiven adhäsiven Acrylmasse und einem oder mehreren Wasser/Feuchtigkeit absorbierenden Wasser/Feuchtigkeit leitenden Substanzen. Das zweite Dokument beschreibt ein Pflaster für einen Wundverschluß mit einer selbstklebenden Matrix mit 2-20 Gew.-% Feuchtigkeit absorbierenden Komponenten in Form von wasserlöslichen und quellbaren synthetischen Kohlenwasserstoffen oder natürlichen Hydrocolloiden, ausgewählt aus einer Gruppe bestehend aus Naturgummie, Silicongummi, Acrylonitrilgummi, Polyurethankautschuk, Polyisobutylen und Acrylpolymeren. Die bekannten Dokumente zeigen unter anderem, daß unterschiedliche Formulierungen einer selbstklebenden Matrix mit unterschiedlichen Wasserabsorbern höchst unterschiedliche Wirkungen bezüglich Wasser-/Feuchtigkeitsabsorption zeigen.

Überraschenderweise wurde nun gefunden, daß die der Erfindung zugrundeliegende Aufgabe dadurch gelöst werden kann, daß man den polymeren Wirkstoffträger möglichst polar macht und einen zusätzlichen, sogenannten Wasserabsorber zusetzt.

Als relativ polares selbstklebendes Grundpolymer wurde ein Polyacrylat verwendet, da diese Kleberklasse vielfältige Anwendungsmöglichkeiten im medizinischen Bereich gefunden hat und als gut hautverträglich gilt. Als besonders gut geeignet erwies sich dabei der Polyacrylatkleber Durotak 280-2516 der Fa. National Starch.
Als polare nichtklebende Polyacrylate kommen solche in Betracht, die einen gewissen Gehalt an folgenden freien polaren Gruppen aufweisen: Hydroxygruppen, Carboxylgruppen, Aminogruppen, quartäre Ammoniumgruppen etc.
Gut eingesetzt für diesen Zweck können z.B. die Polyacrylate der Eudragit Reihe der Firma Röhm-Pharma, da sie breite Anwendung in der Tablettentechnologie gefunden haben und als physiologisch unbedenklich angesehen werden können. Besonders gut geeignet ist Eudragit RL 100, das chemisch als ein Copolymer aus Acryl- und Methacrylsäureestern mit einem gewissen Gehalt an quartären Ammoniumgruppen bezeichnet werden kann. Es zeichnet sich dadurch aus, daß es weitgehend pH-Wert unabhängig quillt und schon dadurch die Aufnahme von Feuchtigkeit in das System fördert. Desweiteren kompensiert es etwas die weichmachende Wirkung von zusätzlichen Hilfsstoffen wie z.B. 1,2-Propandiol.

Als Wasserabsorber werden auf dem Markt eine Vielzahl von Produkten auf der Basis von natürlichen und synthetischen Polymeren angeboten. Als geeigent haben sich Wasserabsorber auf der Basis von leicht quervernetzten, vorneutralisierten Polyacrylsäuren erwiesen. Die besten Resultate wurden erzielt mit dem Produkt Aquakeep 10 SH, der Fa. Seitetsu Kagaku. Aufgrund ihrer Quervernetzung lösen sich solche Wasserabsorber natürlich nicht homogen in der Pflastermatrix, haben aber in der notwendigen Menge keinen negativen Einfluß auf die Kohäsion und die Klebkraft der Matrix.

Die Rückschicht kann aus flexiblem oder nicht flexiblem Material bestehen und ein- oder mehrschichtig ausgestattet sein. Substanzen, die zu ihrer Herstellung verwendet werden können, sind polymere Substanzen, wie z.B. Polyethylen, Polypropylen, Polyvinylchlorid, Polyethylenterephthalat und Polyamid Als weitere Materialien können auch Metallfolien, wie Aluminiumfolie, allein oder mit einem polymeren Substrat beschichtet, angewandt werden. Eine bevorzugte Ausführung ist eine 10µ dicke Polyethylenterephthalatfolie, die matrixseitig aluminisiert und auf der nach Applikation nach außen gewandten Seite hautfarben eingefärbt ist.

Die ablösbare Schutzschicht, die mit der selbstklebenden Matrix in Berührung steht und vor der Anwendung entfernt wird, besteht beispielsweise aus denselben Materialien wie sie zur Herstellung der Deckschicht benutzt werden, vorausgesetzt, daß sie ablösbar gemacht werden, wie z.B. durch eine Siliconbehandlung. Andere ablösbare Schutzschichten sind z.B. aus Polytetrafluorethylen, behandeltem Papier, Cellophan u.ä.

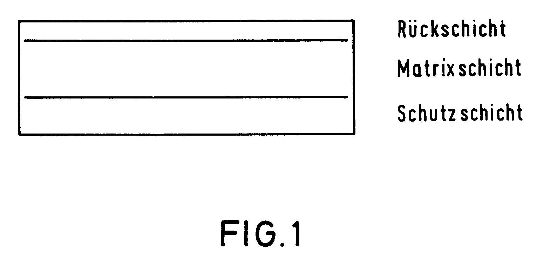

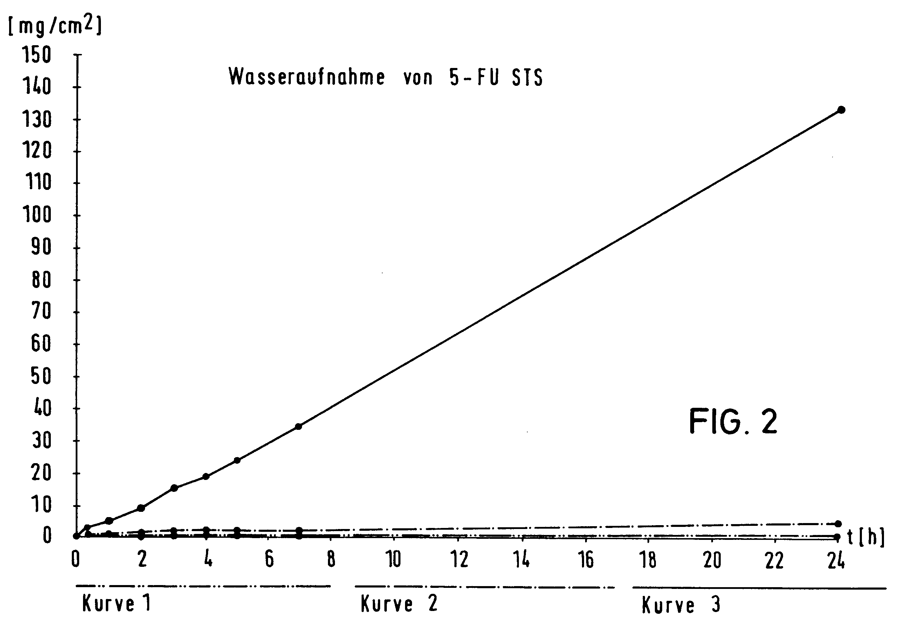

Ein System im Sinne dieser Erfindung zeigt Figur 1 im Querschnitt. Figur 2 demonstriert die Steigerung der Wasseraufnahme des Acrylatklebers durch den Zusatz von Eudragit RL 100 und Aquakeep 10 SH. Kurve 1 zeigt die praktisch zu vernachlässigende Wasseraufnahme des reinen Acrylatklebers, Kurve 2 die leichte Steigerung durch den Zusatz von Eudragit RL 100 und Kurve 3 das dramatische Ansteigen durch den Zusatz von Aquakeep 10 SH.

Kurve 3 liegt folgende sich nach Entfernung des Lösemittels ergebende Matrixformulierung zugrunde, die sich auch im klinischen Versuch als sehr gut wirksam erwiesen hat:
7910 g Polyacrylatkleber (Durotak 280-2516, Fa. National Starch)
1980 g Copolymer aus Acryl- und Methacrylsäureestern mit einem gewissen Gehalt an quartären Ammoniumgruppen (Eudragit RL 100, Fa. Röhm-Pharma)
500 g Wasserabsorber auf Basis von quervernetzter neutralisierter Polyacrylsäure (Aquakeep 10 SH, Fa. Seitetsu Kagaku)
1040 g 1,2-Propandiol
85 g 5-Fluoruracil
Flächengewicht: 115 g/m²

Kurve 2 und 1 liegt die gleiche Formulierung und das gleiche Flächengewicht allerdings ohne Aquakeep 10 SH bzw. ohne Aquakeep 10 SH und Eudragit RL 100 zugrunde.

Die Messungen wurden bei 32°C mit dem. Wasser durchgeführt und die Wasseraufnahme gravimetrisch bestimmt.

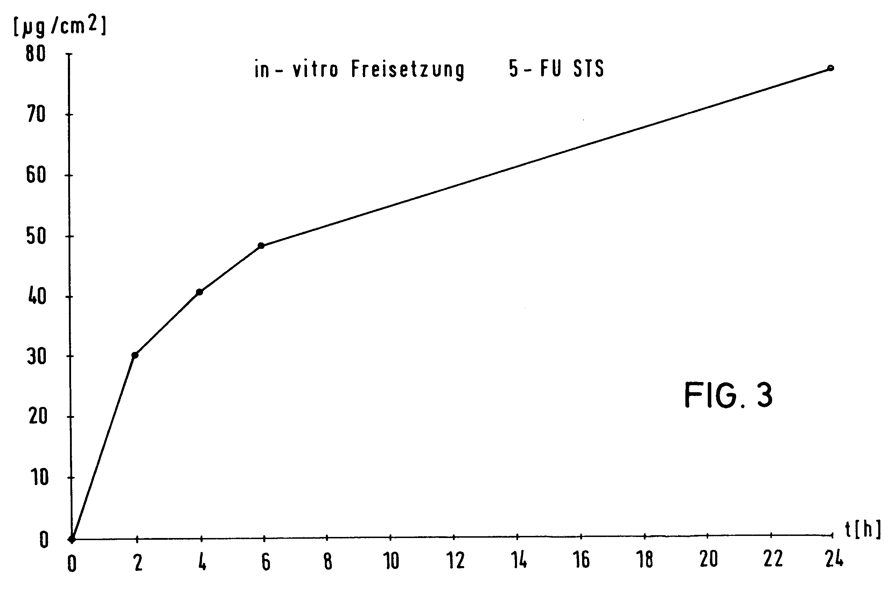

In Figur 3 ist die in-vitro Freisetzung eines Musters auf der Basis obiger Formulierung dargestellt. Der Wirkstoffgehalt beträgt 85 µg/cm². Die Freisetzungskurve zeigt einen Verlauf wie er für Matrixsysteme typisch ist.

Die Freisetzung wurde mittels einer "Rotating Bottle" Apparatur bei 32°C unter Verwendung von physiologischer Kochsalzlösung als Freisetzungsmedium durchgeführt, und die Wirkstoffkonzentration in den Probenlösungen photometrisch gemessen.

Mit Systemen gleicher Matrixformulierung, kreisförmiger Geometrie und einer Größe von 1,13 cm² wurden Klinikversuche an 8 Patienten mit der Indikation aktinische Keratose durchgeführt und in allen Fällen war nach der Applikation von 6-7 Systemen ein Therapieerfolg zu verzeichnen. Der Wechsel der Systeme wurde alle 2 bis 3 Tage vorgenommen.

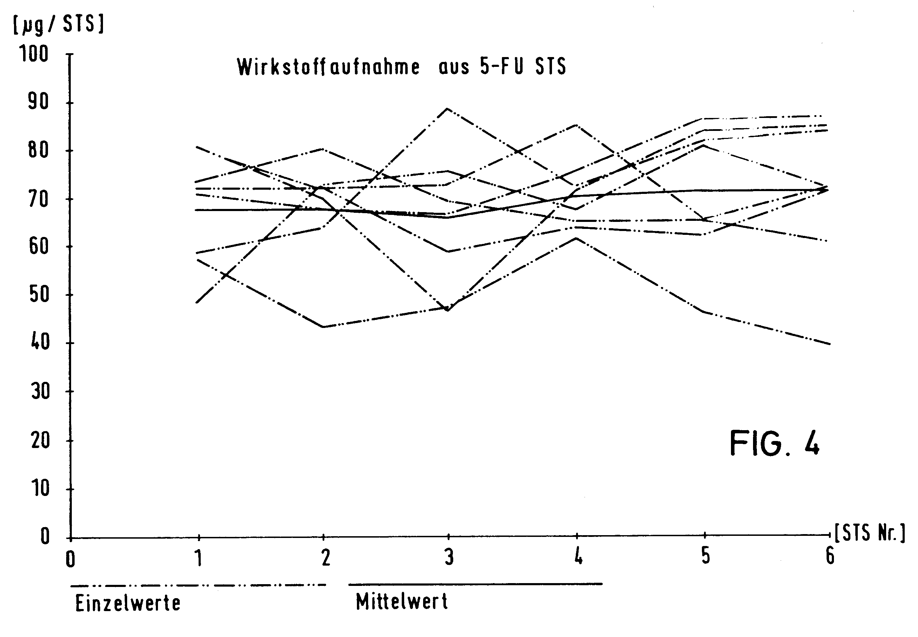

In Figur 4 ist die Aufnahme von 5-Fluoruracil aus den Systemen gezeigt, wie sie durch Rückstandsbestimmung des Wirkstoffs in den getragenenen Systemen bestimmt wurde.

Im Mittel (berechnet aus den Versuchen an 8 Patienten und je 6 Systemen pro Patient) wurden 69,5% bzw. 63,8 µg der eingearbeiteten 91,8 µg 5-Fluoruracil während der Tragezeit der Systeme von 2 oder 3 Tagen aufgenommen. Dies entspricht einer durchschnittlichen Wirkstoffaufnahme von ca. 30 µg 5-Fluoruracil pro Patient, Tag und System. Bei dieser außerordentlich geringen Wirkstoffaufnahme sind systemisch toxische Nebenwirkungen mit Sicherheit auszuschließen.

Die besonderen Vorteile der Erfindung seien untenstehend nochmals zusammengefaßt:
a. sichere therapeutische Wirkung bei minimaler Wirkstoffaufnahme
b. kurze Behandlungsdauer
c. der Wirkstoff wird scharf begrenzt lediglich auf die zu behandelnde Fläche appliziert
d. hohe Wasseraufnahmekapazität des Systems
e. phototoxische Reaktionen werden durch die Okklusionsbedingungen unterdrückt
f. gute kosmetische Resultate
g. wesentlich verbesserte Patientencompliance durch die nur alle 2-3 Tage erforderliche Applikation eines neuen Systems (Salbe 2 x täglich).

### Beispiel

### Herstellungsverfahren für ca. 100 m² eines 5-Fluoruracil superfiziellen therapeutischen Systems

4.352 g einer 40%-igen (G/G)Lösung eines Copolymers aus Acryl- und Methacrylsäureestern mit einem gewissen Gehalt an quartären Ammoniumgruppen (Eudragit RL 100, Fa. Röhm Pharma) in Methylethylketon werden unter Rühren zu 16.697,8 g einer 42%igen (G/G)Lösung eines Polyacrylatklebers (Durotak 280-2516, Fa. National Starch) gegeben, 436 g Wasserabsorber auf Basis von quervernetzter neutralisierter Polyacrylsäure (Aquakeep 10 SH, Fa. Seitetsu Kagaku, Teilchengröße 125µm) und anschließend eine Lösung von 75 g 5-Fluoruracil in 2.753 g 1,2-Propandiol zugefügt.

Diese Masse wird auf eine 100 µ dicke aluminisierte und silikonisierte Polyesterfolie gestrichen, so daß nach dem Entfernen des Lösemittels ein Film mit einem Flächengewicht von 115 g/m² resultiert. Dieser Film wird mit einer 10 µ dicken Polyesterfolie abgedeckt und in Stücke der gewünschten Größe geschnitten und gestanzt.

## Patentansprüche (Patentansprüche für folgende(n) Vertragsstaat(en): AT, BE, CH, DE, DK, FR, GB, IT, LI, LU, NL, SE)

1. Superfizielles therapeutisches System bestehend aus einer undurchlässigen Rückschicht, einer wirkstoffhaltigen Matrix und einer wiederablösbaren Schutzschicht, dadurch gekennzeichnet, daß die Matrix enthält:
a. 0,2 bis 5%, bevorzugt 0,3 bis 1% und besonders bevorzugt 0,6 bis 0,9% antineoplastischen Wirkstoff,
b. mindestens 50%, bevorzugt 65 bis 75% eines polaren polymeren Wirkstoffträgers in Form eines selbstklebenden Polyacrylates,
c. 1 bis 15%, bevorzugt 1 bis 10% und besonders bevorzugt 4 bis 5% eines dem Wirkstoff zugesetzten Wasserabsorbers auf der Basis von quervernetzten, vorneutralisierten Polyacrylsäuren, und gegebenenfalls
d. 0 bis 48,8%, bevorzugt 10 bis 35% und besonders bevorzugt 15 bis 25% eines nichtklebenden hydrophilen Polyacrylats auf der Basis eines Copolymers aus Acryl- und Methacrylsäureestern mit einem Gehalt an quartären Ammoniumgruppen
e. 0 bis 20, bevorzugt 5 bis 15% und besonders bevorzugt 5 bis 10% eines Weichmachers oder Penetrationsbeschleunigers.

2. Superfizielles therapeutisches System gemäß Anspruch 1, dadurch gekennzeichnet, daß der antineoplastische Wirkstoff 5-Fluoruracil ist.

3. Superfizielles therapeutisches System gemäß einem der Ansprüche 1 oder 2, dadurch gekennzeichnet, daß der Weichmacher bzw. Penetrationsbeschleuniger 1,2-Propandiol ist.

4. Superfizielles therapeutisches System gemäß einem der Ansprüche 1 bis 3, gekennzeichnet durch eine kreisförmige Geometrie und einen Durchmesser von 0,5 bis 3 cm, bevorzugt von 1 bis 2 cm, und besonders bevorzugt von 1 bis 1,2 cm.

5. Superfizielles therapeutisches System gemäß einem der Ansprüche 1 bis 3, dadurch gekennzeichnet, daß es rechtekkig ist und eine Fläche von 1 bis 200 cm², bevorzugt 1 bis 50, und besonders bevorzugt von 2 bis 20 cm² besitzt.

## Patentansprüche (Patentansprüche für folgende(n) Vertragsstaat(en): ES, GR)

1. Verfahren zur Herstellung eines superfiziellen therapeutischen Systems, bestehend aus einer undurchlässigen Rückschicht, einer wirkstoffhaltigen Matrix und einer wieder ablösbaren Schutzschicht, dadurch gekennzeichnet, daß die folgenden Bestandteile der Matrix
a. 0,2 bis 5%, bevorzugt 0,3 bis 1% und besonders bevorzugt 0,6 bis 0,9% antineoplastischer Wirkstoff,
b. mindestens 50%, bevorzugt 65 bis 75% eines polaren polymeren Wirkstoffträgers in Form eines selbstklebenden Polyacrylates,
c. 1 bis 15%, bevorzugt 1 bis 10% und besonders bevorzugt 4 bis 5% eines dem Wirkstoff zugesetzten Wasserabsorbers auf der Basis von quervernetzten, vorneutralisierten Polyacrylsäuren, und gegebenenfalls
d. 0 bis 48,8%, bevorzugt 10 bis 35% und besonders bevorzugt 15 bis 25% eines nichtklebenden hydrophilen Polyacrylats auf der Basis eines Copolymers aus Acryl- und Methacrylsäureestern mit einem Gehalt an quartären Ammoniumgruppen, sowie
e. 0 bis 20%, bevorzugt 5 bis 15% und besonders bevorzugt 5 bis 10% eines Weichmachers oder Penetrationsbeschleunigers
homogen, gegebenenfalls durch Auflösen in einem Lösungsmittel, vermischt, die homogene Mischung auf die undurchlässige Rückschicht aufgebracht, gegebenenfalls das Lösungsmittel entfernt und danach die Matrixschicht mit einer Schutzschicht abgedeckt wird.

2. Verfahren gemäß Anspruch 1, dadurch gekennzeichnet, daß der antineoplastische Wirkstoff 5-Fluoruracil ist.

3. Verfahren gemäß einem der Ansprüche 1 oder 2, dadurch gekennzeichnet, daß der Weichmacher bzw. Penetrationsbeschleuniger 1,2-Propandiol ist.

4. Verfahren gemäß einem der Ansprüche 1 oder 2, dadurch gekennzeichnet, daß das System nach dem Abdecken mit der wiederablösbaren Schutzschicht zu kreisförmigen Stücken mit einem Durchmesser von 0,5-3 cm, bevorzugt von 1-2 cm, und besonders bevorzugt von 1-1,2 cm zugeschnitten wird.

5. Verfahren gemäß einem der Ansprüche 1 oder 2, dadurch gekennzeichnet, daß das System nach Abdeckung mit der Schutzfolie auf Rechtecksform mit einer Fläche von 1 bis 200 cm², bevorzugt 1-50, und besonders bevorzugt von 2-20 cm² zugeschnitten wird.

## Claims (Claims for the following Contracting State(s): AT, BE, CH, DE, DK, FR, GB, IT, LI, LU, NL, SE)

1. Superficial therapeutic system consisting of an impermeable backing layer, an active substance containing matrix and a removable protective layer, characterized in that the matrix comprises:
a. 0.2-5%, preferably 0.3-1%, and particularly preferred 0.6-0.9%, of an antineoplastic active substance,
b. at least 50%, preferably 65-75%, of a polar polymeric active substance carrier in the form of a self-adhesive polyacrylate
C. 1-15%, preferably 1-10%, and particularly preferred 4-5%, of a water absorber added to the active substance, said water absorber being based on cross-linked, pre-neutralized polyacrylic acids, and, optionally,
d. 0-48.8%, preferably 10-35%, and particularly preferred 15-25%, of a non-adhesive hydrophilic polyacrylate based on a copolymer of acrylic acid esters and methacrylic acid esters containing a quaternary ammonium group,
e. 0-20%, preferably 5-15%, and particularly preferred 5-10%, of a softener or penetration accelerator.

2. The superficial therapeutic system according to claim 1, characterized in that the antineoplastic active substance is 5-fluorouracil.

3. The superficial therapeutic system according to one of the claims 1 or 2, characterized in that the softener or penetration enhancer, respectively, is 1,2-propanediol

4. The superficial therapeutic system according to any one of claims 1 to 3, characterized by a circular shape and a diametre of 0.5-3 cm, preferably 1-2 cm, and particularly preferred 1-1.2 cm.

5. The superficial therapeutic system according to any one of claims 1-3, characterized in that it is rectangular and has an area of 1-200 cm², preferably 1-50, and particularly preferred 2-20 cm².

## Claims (Claims for the following Contracting State(s): ES, GR)

1. A process for the production of a superficial therapeutic system consisting of an impermeable backing layer, an active substance containing matrix and a removable protective layer, characterized in that the following components of the matrix
a. 0.2-5%, preferably 0.3-1%, and particularly preferred 0.6-0.9%, of an antineoplastic active substance,
b. at least 50%, preferably 65-75%, of a polar polymeric active substance carrier in the form of a self-adhesive polyacrylate
C. 1-15%, preferably 1-10%, and particularly preferred 4-5%, of a water absorber added to the active substance, said water absorber being based on cross-linked, pre-neutralized polyacrylic acids, and, optionally,
d. 0-48.8%, preferably 10-35%, and particularly preferred 15-25%, of a non-adhesive hydrophilic polyacrylate based on a copolymer of acrylic acid esters and methacrylic acid esters containing a quaternary ammonium group,
e. 0-20%, preferably 5-15%, and particularly preferred 5-10%, of a softener or penetration accelerator.
are mixed, optionally by dissolution in a solvent, the homogenous mixture is applied to the impermeable backing layer, the solvent is optionally removed and, subsequently, the matrix layer is covered with a protective layer.

2. The process according to claim 1, characterized in that the antineoplastic active substance is 5-fluorouracil.

3. The process according to claim 1 or 2, characterized in that the softener or penetration enhancer, respectively, is 1,2-propanediol

4. The process according to claim 1 or 2, characterized in that the system, subsequent to covering with the removable protective layer, is cut to circular pieces having a diametre of 0.5-3 cm, preferably 1-2 cm, and particularly preferred 1-1.2 cm.

5. The process according to claim 1 or 2, characterized in that the system, subsequent to covering with the protective layer, is cut to a rectangular shape having an area of 1-200 cm², preferably 1-50, and particularly preferred 2-20 cm²

## Revendications (Revendications pour l'(les) Etat(s) contractant(s) suivant(s): AT, BE, CH, DE, DK, FR, GB, IT, LI, LU, NL, SE)

1. Système thérapeutique superficiel, constitué d'une couche dorsale imperméable, d'une matrice contenant un principe actif et d'une couche de protection amovible, caractérisé en ce que la matrice contient :
a. 0,2 à 5%, de préférence 0,3 à 1% et, plus spécialement 0,6 à 0,9%, d'un principe antinéoplasique;
b. au moins 50%, de préférence 65 à 75%, d'un support de principe actif polymérique et polaire sous forme d'un polyacrylate autocollant;
c. 1 à 15%, de préférence 1 à 10% et, plus particulièrement, 4 à 5% d'un agent d'absorption d'eau ajouté au principe actif, à base de poly(acides acryliques) préneutralisés et réticulés et, éventuellement:
d. 0 à 48,8%, de préférence 10 à 35% et, plus particulièrement, 15 à 25%, d'un polyacrylate hydrophile et non collant, à base d'un copolymère d'esters de l'acide acrylique et de l'acide méthacrylique avec une teneur en radicaux ammonium quaternaire;
e. 0 à 20%, de préférence 5 à 15% et, plus particulièrement, 5 à 10%, d'un plastifiant ou ramollissant, ou d'un accélérateur de pénétration.

2. Système thérapeutique superficiel selon la revendication 1, caractérisé en ce que le principe antinéoplasique est le 5-fluoruracile.

3. Système thérapeutique superficiel selon l'une quelconque des revendications 1 ou 2, caractérisé en ce que le plastifiant ou ramollissant ou l'accélérateur de pénétration est le 1,2-propanediol.

4. Système thérapeutique superficiel selon l'une quelconque des revendications 1 à 3, caractérisé par une géométrie circulaire et un diamètre de 0,5 à 3 cm, de préférence de 1 à 2 cm et, plus particulièrement, de 1 à 1,2 cm.

5. Système thérapeutique superficiel selon l'une quelconque des revendications 1 à 3, caractérisé en ce qu'il est rectangulaire et possède une surface de 1 à 200 cm², de préférence 1 à 50 cm² et, plus particulièrement, de 2 à 20 cm².

## Revendications (Revendications pour l'(les) Etat(s) contractant(s) suivant(s): ES, GR)

1. Procédé de préparation d'un système thérapeutique superficiel, constitué d'une couche dorsale imperméable, d'une matrice contenant un principe actif et d'une couche de protection amovible, caractérisé en ce que l'on mélange les constituants suivants de la matrice :
a. 0,2 à 5%, de préférence 0,3 à 1% et, plus spécialement 0,6 à 0,9%, d'un principe antinéoplasique;
b. au moins 50%, de préférence 65 à 75%, d'un support de principe actif polymérique et polaire sous forme d'un polyacrylate autocollant;
c. 1 à 15%, de préférence 1 à 10% et, plus particulièrement, 4 à 5% d'un agent d'absorption d'eau ajouté au principe actif, à base de poly(acides acryliques) préneutralisés et réticulés et, éventuellement:
d. 0 à 48,8%, de préférence 10 à 35% et, plus particulièrement, 15 à 25%, d'un polyacrylate hydrophile et non collant, à base d'un copolymère d'esters de l'acide acrylique et de l'acide méthacrylique avec une teneur en radicaux ammonium quaternaire;
e. 0 à 20%, de préférence 5 à 15% et, plus particulièrement, 5 à 10%, d'un plastifiant ou ramollissant, ou d'un accélérateur de pénétration,
de manière homogène, éventuellement par dissolution dans un solvant, on applique le mélange homogène sur la couche dorsale imperméable, on élimine éventuellement le solvant et on recouvre ensuite la couche formant la matrice d'une couche de protection.

2. Procédé selon la revendication 1, caractérisé en ce que le principe antinéoplasique est le 5-fluoruracile.

3. Procédé selon l'une quelconque des revendications 1 ou 2, caractérisé en ce que le plastifiant ou ramollissant ou l'accélérateur de pénétration est le 1,2-propanediol.

4. Procédé selon l'une quelconque des revendications 1 ou 2, caractérisé en ce qu'après le recouvrement par la couche de protection amovible, on découpe le système en pièces circulaires d'un diamètre de 0,5 à 3 cm, de préférence de 1 à 2 cm et, plus particulièrement, de 1 à 1,2 cm.

5. Procédé selon l'une quelconque des revendications 1 ou 2, caractérisé en ce qu'après le recouvrement avec la feuille de protection, on découpe le système sous forme rectangulaire d'une surface de 1 à 200 cm², de préférence 1 à 50 cm² et, plus particulièrement, de 2 à 20 cm².
